# EUROPEAN PATENT APPLICATION

(11) **EP 4 082 537 A1**
(43) Date of publication of application: **02.11.2022**
(21) Application number: 21185632.3
(22) Date of filing: 14.07.2021
(51) Int. Cl.: A61K 31/155, A61K 31/165, A61P 3/00, A61P 3/04

(54) **ANTI-OBESITY COMPOSITION CONTAINING COLCHICINE AND METFORMIN AS EFFECTIVE AGENTS**

(30) Priority: 29.04.2021 KR 20210055801
(71) Applicant: Acebiomed, Inc., Jinju, Gyeongnam 52893 (KR)
(72) Inventor: YOUN, Ju Ho, Seoul (KR); HAM, Sun Ah, Gyeongsangnam-do (KR)
(74) Representative: Prüfer & Partner mbB Patentanwälte · Rechtsanwälte

(57) **Abstract**

The present invention relates to a complex formulation for treating adipositas or improving a therapeutic effect, and more particularly, to a complex formulation prepared by including colchicine and metformin as main effective agents. According to the present invention, the composition may be used as an excellent pharmaceutical composition capable of preventing or treating obesity by efficiently decomposing fat accumulated in the body by a synergic effect of colchicine and metformin.

## Description

### [Technical Field]

The present invention relates to a complex formulation for treating adipositas or improving a therapeutic effect, wherein the complex formulation is prepared by including colchicine and metformin as main effective agents. More specifically, the present invention relates to a pharmaceutical composition which exhibits a more pharmaceutical effect than drugs to be administered alone in the related art when co-administering colchicine and metformin known as conventional therapeutic agents for liver disease, etc. to patients with adipositas, and relates to a pharmaceutical composition for treating anti-obesity prepared by specifically limiting the contents of the drugs.

### [Background Art]

Obesity or adipositas is a disease in which fat is excessively accumulated in the human body. The obesity is a biological phenomenon caused by the interaction of genetic, metabolic, environmental, and behavioral complex factors, and has generally been recognized as overweight. Medically, when a body mass index (BMI) is 30 or more (i.e., 30% or more of a standard body weight) or the BMI is 27 or more, it is classified as obesity, and in modern people, overweight and obesity are increasing due to the westernized diet and the small amount of exercise.

In the world health organization (WHO), when the BMI is 30 or more, it is referred to as adipositas. Usually, in the case of the obesity, not only the body weight of obese people is recognized as being heavier than a normal value, but also the body fat percentage of the components of the body is high.

Further, according to the WHO, it has been reported that more than a billion adults worldwide are overweight, at least 3 million among them are clinically obese, and more than 25,000 people die each year from overweight-related diseases. Particularly, the obesity is known as an important factor that causes various adult diseases, such as hypertension, Type 2 diabetes, cancer, gallbladder disease, hyperlipidemia, atherosclerosis, etc., which consequently brings about a decrease in expected sleep.

It is difficult to see the cause of obesity only as a genetic cause, and there is a widespread perception that genetic and environmental complex factors that destroy the energy balance are important causes of obesity.

The obesity itself may cause apparent problems such as psoriasis and stretchmarks and social disorders, and above all, secondary complications caused by excess fat are fatal to health.

In addition, the obesity is an important factor in metabolic diseases, and a direct association with metabolic diseases and stroke, myocardial infarction and cardiovascular disease is well known. Therefore, the obesity causes many metabolic disorders to be finally closely related to cardiovascular and cerebrovascular disorders. That is, the obesity itself, which causes various medical problems that inhibit health and an increase in early death, is one of important chronic diseases and the most important threat of health in the modern society.

It is most preferable to first emphasize non-drug therapy through lifestyle modification as a treatment method of obesity. However, despite these treatments, if the weight loss is not successful, medication treatment and surgical treatment are considered. As a drug treatment method of diabetes, in Korea, the market of anorectic agents classified as psychotropic drugs has been greatly expanded. The scale of the psychotropic anorectic agents or imported drugs without ingredients for weight loss has not even been determined. At one time, in Japan, deaths from the intake of Chinese diet foods were reported, and a request for attention of the intake of Chinese diet foods was notified. In addition, safety problems according to the overuse, dependence and drug abuse of psychotropic drugs are steadily raised and thus, compliance of use-by dates and careful co-administration of these drugs are required.

Accordingly, a lot of attention has been focused on the development of new effective obesity therapeutic agents that can overcome the limitations of conventional obesity therapeutic agents. However, the neurobiological aspects of achieving the pathophysiology of obesity are very complex, and this complexity means that there is a little possibility of causing dramatic weight loss by targeting only one path. That is, for continuously meaningful weight loss, the development of various drugs that act on different mechanisms is required.

Obesity therapeutic agents may be divided into drugs that act on the central nervous system to affect appetite, heat producing accelerators that act on the central nervous system or the periphery, and drugs that act on the gastrointestinal system to reduce absorption.

Most anorectic agents applied to the treatment of obesity are psychotropic anorectic agents that cause appetite suppression by increasing the efficacy of appetite-controlled nerve control materials, such as norepinephrine, serotonin, dopamine, etc. in the brain.

Currently, in obesity drugs usable in Korea, as agents approved for short-term use, there are phentermine, diethylpropion, phendimetrazine, and mazindol, and as agents approved for long-term use, there are orlistat, naltrexone ER/bupropion ER (NB), phentermine/topiramate CR (PHEN/TPM CR), and liraglutide.

Since some of the drugs have a risk of tumor occurrence and there is also a possibility of increasing the body weight depending on a current condition of a patient, selecting a drug for treating obesity should be determined after a close analysis of health conditions and a drug intake state of a patient.

As such, most of conventional obesity therapeutic agents may affect the health of the patient as a psychotropic agent. Accordingly, the present inventors developed a drug of combining colchicine for treating liver disease, known as a stable ingredient in the human body and metformin known as a drug for treating hyperlipidemia while developing a drug capable of solving these problems to develop a complex formulation capable of treating obesity without affecting the health condition of the patient, and then completed the present invention.

### [Prior Arts]

### [Patent Document]

Korean Patent Publication No. 10-2020-0104238
Korean Patent Publication No. 10-2009-0108230

### [Disclosure]

### [Technical Problem]

The present invention has been developed to solve the problems of conventional obesity therapeutic agents as described above, and may solve obesity caused by fat, etc. without problems such as psychotropic for a patient, when co-administering colchicine widely used as a therapeutic agent for liver disease, gout-related arthritis, or the like and metformin used as a therapeutic agent for hyperlipidemia or the like, as a complex formulation for treating obesity.

In addition, the present invention is to provide a dose exhibiting the most suitable efficacy when co-administering the colchicine and the metformin in the human body.

The term "obesity" of the present invention refers to a condition or disease with excessive body fat by energy imbalance. It is possible to prevent or treat obesity by administering a pharmaceutical composition according to the present invention to a subject to lose the body weight.

The term "prevention" of the present invention means all actions that inhibit or delay the onset of the obesity by administration of the pharmaceutical composition according to the present invention.

The term "treatment" of the present invention means all actions that improve or beneficially change the symptoms of obesity by administering the pharmaceutical composition of the present invention.

The technical objects to be solved by the present invention are not limited to the aforementioned objects, and various objects associated with the objects can be clearly understood to those skilled in the art to which the present invention pertains from the following description.

### [Technical Solution]

In order to achieve the objects, the present invention provides a pharmaceutical composition for treating adipositas comprising colchicine and metformin as main effective agents.

Further, the present invention provides a pharmaceutical composition for improving an obesity therapeutic effect comprising colchicine and metformin as main effective agents. The pharmaceutical composition for improving the therapeutic effect is the same concept as a therapeutic adjuvant for preventing and treating adipositas, a target disease of the present invention.

The pharmaceutical composition of the present invention may be prepared in the form of a pharmaceutical composition for treating or preventing obesity further comprising suitable carriers, excipients, or diluents which are commonly used for the preparation of the pharmaceutical composition, wherein the carriers may include a non-naturally occurring carrier. Specifically, the pharmaceutical composition may be formulated and used in the form of oral formulations such as powders, granules, tablets, capsules, suspensions, emulsions, syrups, aerosols, etc., external preparations, suppositories, and sterile injectable solutions according to a general method.

In the present invention, the carriers, the excipients, and the diluents, which may be included in the pharmaceutical composition, may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In the case of the formulations, the formulation may be prepared by using a diluent or an excipient, such as a filler, an extender, a binder, a wetting agent, a disintegrating agent, and a surfactant which are generally used. Solid formulations for oral administration include tablets, pills, powders, granules, capsules, and the like, and the solid formulation may be prepared by mixing at least one excipient, for example, starch, calcium carbonate, sucrose or lactose, gelatin, and the like. Further, lubricants such as magnesium stearate, talc may be used in addition to simple excipients. Liquid formulations for oral administration may correspond to suspensions, oral liquids, emulsions, syrups, and the like, and may include various excipients, for example, a wetting agent, a sweetener, an aromatic agent, a preservative, and the like, in addition to water and liquid paraffin which are commonly used as simple diluents. Formulations for parenteral administration include sterilized aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

The pharmaceutical composition of the present invention may be administered in a pharmaceutically effective dose, and the term "pharmaceutically effective dose" herein means an amount enough to treat or prevent a disease at a reasonable benefit/risk ratio applicable to medical treatment or prevention. The effective dose level may be determined depending on factors including the severity of a disease, the activity of a drug, the age, body weight, health, and gender of a patient, the sensitivity to the drug of the patient, the administration time, administration route, emission rate, and treatment period of the composition of the present invention used, combinations with the composition of the present invention used or simultaneously used drugs, and other factors well-known in a medical field. The pharmaceutical composition of the present invention may be administered as an individual therapeutic agent or administered in combination with other therapeutic agents, and sequentially or simultaneously administered with conventional therapeutic agents. In addition, the pharmaceutical composition may be administered singly or multiply. Considering all of the factors, it is important to administer an amount capable of obtaining a maximum effect in a minimum amount without side effects.

The dose of the pharmaceutical composition provided in the present invention may be determined by those skilled in the art by considering the purpose of use, the addiction of a disease, the age, body weight, gender, and medical history of a patient, the type of a material to be used as an effective agent, etc. However, the pharmaceutical composition of the present invention may be administered in an amount of 20 to 1000 mg/kg, more preferably 50 to 400 mg/kg a day with respect to the body weight of a mammal including the human, and the administration frequency of the pharmaceutical composition of the present invention is not particularly limited, but the pharmaceutical composition may be administered once to three times a day or administered several times by dividing the dose.

### [Advantageous Effects]

According to the present invention, the pharmaceutical composition comprising colchicine and metformin as effective agents can prevent or treat obesity by effectively decomposing fat accumulated in the body due to a synergic effect of the two ingredients.

The complex formulation provided in the present invention is administered even in a low dose to efficiently improve an effect of preventing or treating obesity, and thus, will also play a major role in the development of related industries such as human health growth and healthcare.

### [Description of Drawings]

FIG. 1 illustrates changes in body weight occurring after administering drugs provided in the present invention to obese rats induced by a high fat diet.
FIG. 2 illustrates feed intakes of rats occurring after administering drugs provided in the present invention to obese rats induced by a high fat diet.
FIG. 3 illustrates epididymal fat weights occurring after administering drugs provided in the present invention to obese rats induced by a high fat diet.
FIGS. 4A and 4B illustrate expression of obesity-related genes occurring after administering drugs provided in the present invention to obese rats induced by a high fat diet.

### [Modes for the Invention]

When a complex formulation formed by comprising colchicine and metformin as effective agents provided in the present invention is administered to obese rats, an effect of improving obesity is confirmed in the obese rats and the present invention has been completed based thereon.

Hereinafter, the present invention will be described in detail.

The colchicine as the main effective agent used herein is a material extracted from a plant of colchicum genus and a drug used mainly as an alternative drug for the gout when a nonsteroidal anti-inflammatory agent does not smoothly exhibit the therapeutic effect on the gout, and is used even for the treatment of familial Mediterranean fever, periceritis, Behcet's disease, etc.

The metformin as another effective agent used herein has a blood glucose improvement effect as a biguanide-based drug and is mainly used as a therapeutic agent for diabetes. The mechanism of metformin is not accurate, but is known to prevent an increase in concentration of cAMP induced by glucagons by inhibiting cellular respiration occurring in mitochondria and activating AMPK and consequently inhibit the activity of PKA to inhibit the production of glucose.

The inventors of the present invention have confirmed through various experiments that therapeutic efficacy for obesity is increased when co-administering colchicine and metformin.

In an embodiment of the present invention, when comparing changes in body weight of rats in the case of alone-administration of colchicine, alone-administration of metformin, and co-administration of colchicine and metformin to obesity-induced rats, a weight loss effect of rats is greatest in the case of co-administration of colchicine and metformin.

Further, in an embodiment of the present invention, when comparing expression levels of obesity-related genes SREBP-1c and FAS (fatty acid synthase) in the case of alone-administration of colchicine, alone-administration of metformin, and co-administration of colchicine and metformin to obesity-induced rats, the expression levels of the genes are reduced greatest in the case of co-administration of colchicine and metformin.

Sterol regulatory element binding proteins (SREBPs) genes are transcription factors that regulate homeopronability and metabolism of lipids, and these genes precisely regulate the expression of enzymes required for synthesis of endogenous cholesterol, fatty acids (FA), triasyl glycerol (TG) and phospholipids.

The SREBPs include three types of 1a, 1c and 2, and it has been known that SREBP-1a and SREBP-1c are mainly involved in the synthesis of fatty acids and triglycerides and SREBP-2 is involved in the cholesterol metabolism. In the liver tissue, the expression of SREBP-1c is predominant and regulates the expression of genes such as FAS, ACC, stearoyl-CaP desaturase, ATP citrate lyase, and malic enzyme associated with the synthesis of triglycerides in hepatocytes.

Hyperinsulinemia due to insulin resistance increases the biosynthesis of fatty acids by increasing the expression of SREBP-1c in the liver and consequently causes the accumulation of triglycerides in liver tissue. The role of SREBP-1c in the insulin resistance and the accumulation of triglycerides in the liver has been proven through animal experiments. Through studies of reporting that when fatty liver lesions are observed in ob/ob mice, as a feature of high obesity and insulin resistance and the SREBP-1 gene is inactivated in obesity-induced rats, the accumulation of triglycerides in the liver tissue is reduced by about 50%, it can be seen that SREBP-1c plays an important role in the occurrence of fatty liver in an insulin-resistant animal model.

The SREBPs are involved in a lot of disease causing processes such as ER stress, inflammation, autophagocytosis and apoptosis, and are known to cause obesity, dyslipidemia, diabetes, non-alcoholic fatty liver disease, etc.

When administering the complex formulation provided in the present invention, the colchicine is added in an amount of preferably 5 to 300 µg/kg, more preferably 50 to 200 µg/kg, and the metformin is added in an amount of preferably 50 to 400 mg/kg, more preferably 100 to 200 mg/kg.

When the content of the colchicine is less than 5 µg/kg, the efficacy of the colchicine is not almost exhibited, and when the content thereof is more than 300 µg/kg, there is a side effect that causes gastrointestinal disorders at the time of taking.

When the content of the metformin is less than 50 mg/kg, the efficacy thereof is not exhibited, and when the content thereof is more than 400 mg/kg, side effects such as abdominal pain, diarrhea, vomiting, etc. are likely to occur.

Further, in the pharmaceutical composition for treating adipositas provided in the present invention, a content ratio of the colchicine and metformin as the effective agents may include 1:1,000 to 1:20,000, more preferably 1:2,000 to 1:5,000 as the content of metformin to the content of colchicine.

The different term 'complex formulation' as used herein means the pharmaceutical composition provided in the present invention.

The composition of the present invention may further include suitable carriers, excipients, and diluents which are commonly used in the preparation of pharmaceutical composition. Further, according to a general method, the pharmaceutical composition may be formulated and used in the form of oral formulations such as powders, granules, tablets, capsules, suspensions, emulsions, syrups, aerosols, etc., external preparations, suppositories, and sterile injectable solutions, and preferably, formulated and used in a unit dosage form suitable for oral administration.

The carriers, the excipients, and the diluents that may be included in the composition may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, etc. In the case of formulating the composition, the formulation may be prepared by using a diluent or an excipient, such as a filler, an extender, a binder, a wetting agent, a disintegrating agent, and a surfactant which are generally used.

Solid formulations for oral administration include tablets, pills, powders, granules, capsules, etc., and these solid formulations may be prepared by mixing at least one excipient, for example, starch, calcium carbonate, sucrose, lactose, gelatin, and the like with the composition. Further, lubricants such as magnesium stearate, and talc may be used in addition to simple excipients. Liquid formulations for oral administration may correspond to suspensions, oral liquids, emulsions, syrups, and the like, and may include various excipients, for example, a wetting agent, a sweetener, an aromatic agent, a preserving agent, and the like, in addition to water and liquid paraffin which are commonly used as simple diluents. Formulations for parenteral administration include sterilized aqueous solutions, non-aqueous solvents, suspensions, emulsions, lyophilized agents, and suppositories. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

In addition, the composition for treating adipositas and the like provided in the present invention may be further added with an antioxidant. The antioxidant may use compounds in vitamin B group such as thiamine (vitamin B1), riboflavin (vitamin B2), niacin (vitamin B3), pantothenic acid (vitamin B5), pyridoxine (vitamin B6), and cobalamin (vitamin B12), vitamin C, vitamin D, vitamin E, N-acetylcysteine (NAC), and the like.

The pharmaceutical composition of the present invention is administered in a pharmaceutically effective dose. In the present invention, the 'pharmaceutically effective dose' refers to a an amount enough to treat the disease at a reasonable benefit/risk ratio applicable to medical treatment. An effective dose level may be determined according to factors including the type and severity of a disease of a patient, the activity of a drug, the sensitivity to a drug, a time of administration, a route of administration, an emission rate, duration of treatment, and simultaneously used drugs, and other factors well-known in the medical field. The pharmaceutical composition according to the present invention may be administered as an individual therapeutic agent or in combination with other therapeutic agents, and may be administered sequentially or simultaneously with conventional therapeutic agents, and may be administered singly or multiply. It is important to administer an amount capable of obtaining a maximum effect with a minimal amount without side effects by considering all the factors, which may be easily determined by those skilled in the art.

The pharmaceutical composition of the present invention may be administered to a subject through various routes. All methods of administration may be expected, and for example, the pharmaceutical composition may be administered by oral, rectal or intravenous, intramuscular, subcutaneous, intrauterine dural or cerebrovascular injections. The pharmaceutical composition of the present invention is determined according to a type of drug as an effective agent in addition to many related factors, such as a disease to be treated, an administration route, the age, gender, and body weight of a patient, and the severity of the disease.

Hereinafter, a preferred embodiment is presented in order to assist understanding of the present invention. However, the following Examples are just provided to more easily understand the present invention, and the contents of the present invention are not limited by the following Examples.

### Example

### Preparation of experimental animals and experimental method

Low fat diet (LFD: containing 10 kcal% fat) or high fat diet (HFD: containing 60 kcal% fat) feed was taken autonomously for 12 weeks in 6-week-old C57BL/6J sp. male rats. The obesity-induced rats due to the intake of the HFD feed additionally took the HFD feed for 12 weeks and were administered with colchicine of 50, 100, and 200 µg/kg or metformin of 200 mg/kg alone or co-administered with the colchicine and the metformin. A control drug, Xenical (orlistat ingredient) was administered alone at 60 mg/kg. All drugs were orally administered once a day.

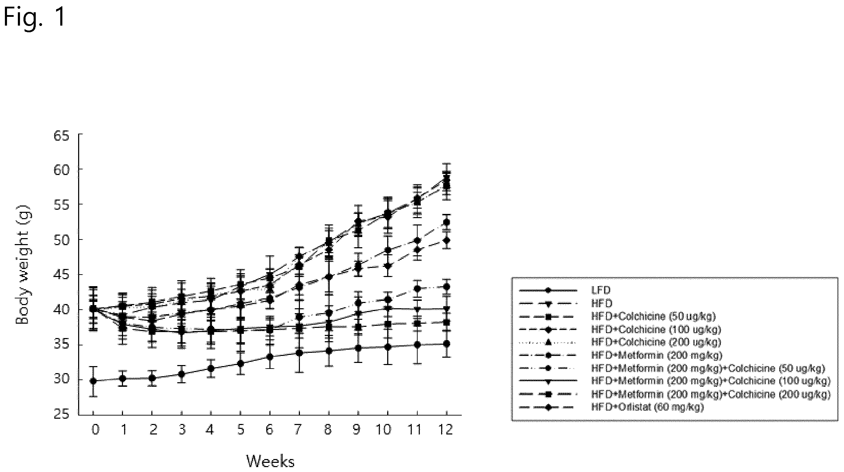

Changes in body weight of obesity-induced rats were measured according to alone-administration of colchicine or metformin and co-administration of colchicine and metformin, which were illustrated in FIG. 1.

As illustrated in FIG. 1, it was confirmed that an increase in body weight induced by the HFD was not changed by alone-administration of colchicine and the body weight was slightly reduced in alone-administration of metformin. In comparison, in co-administration of colchicine and metformin, it was confirmed that the weight loss of obesity-induced rats was much higher than alone-administration of the two ingredients, and particularly, when the content of colchicine was smaller than the content of metformin, a weight loss effect was relatively excellent.

This effect was shown to be more significant than the control drug, Xenical.

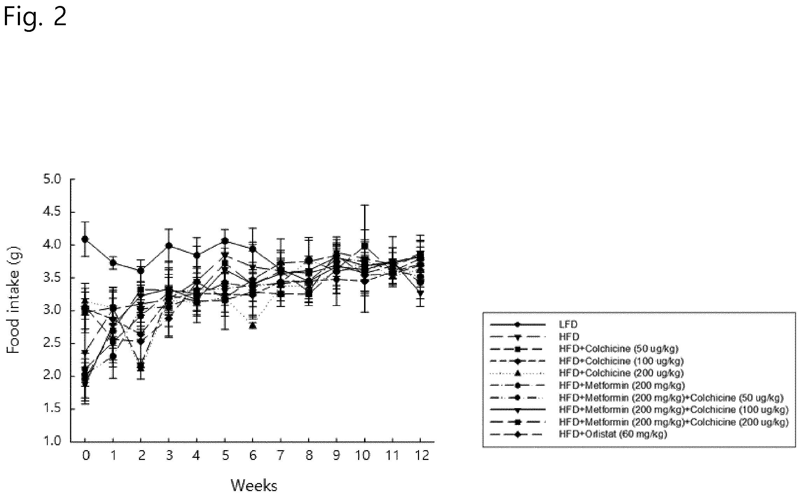

After the drugs were administered to the experimental animals, obesity-induced rats, the intakes of the feed were compared, and as illustrated in FIG. 2, there was no large difference in the intakes of the feed for each drug.

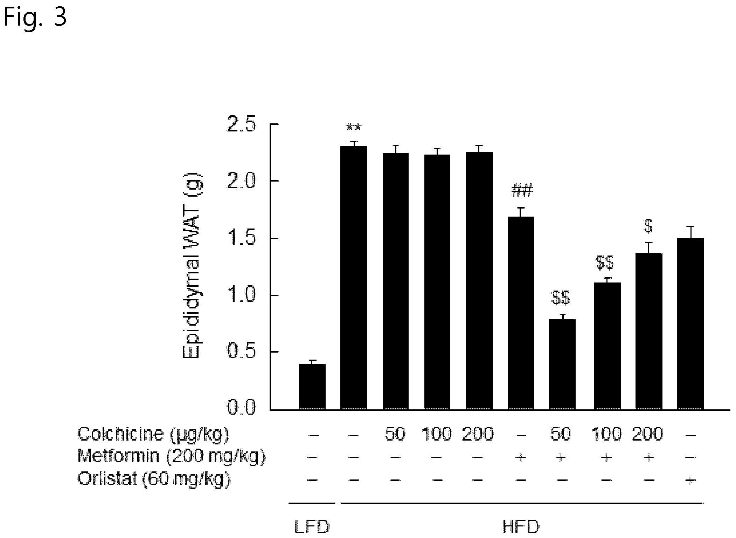

Next, after the drugs were administered to the obesity-induced rats, the weights of epididymal fat were measured and the results thereof was illustrated in FIG. 3.

As illustrated in FIG. 3, it was confirmed that the weight of epididymal fat of obesity-induced rats increased by the HFD was not changed by alone-administration of colchicine and slightly reduced in alone-administration of metformin. In comparison, in co-administration of colchicine and metformin, it was confirmed that the weight of epididymal fat was much reduced, and particularly, when the content of colchicine was smaller than the content of metformin, it was confirmed that the weight loss effect was relatively excellent, and much more reduced than the control drug, Xenical.

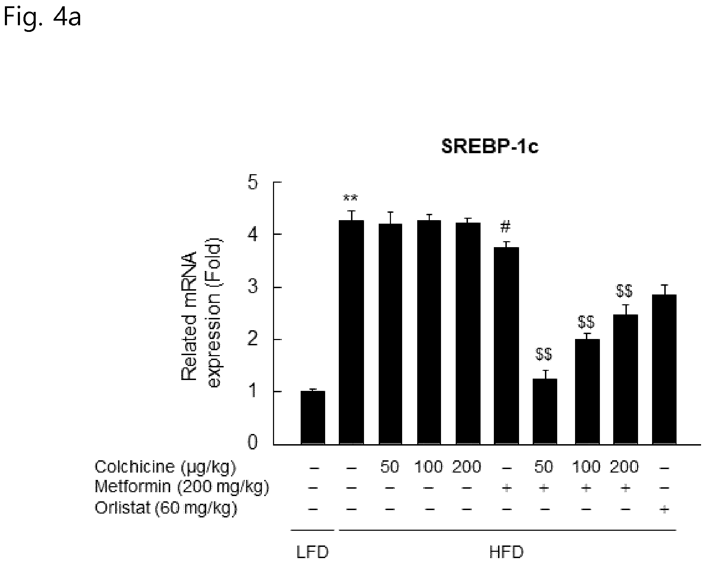

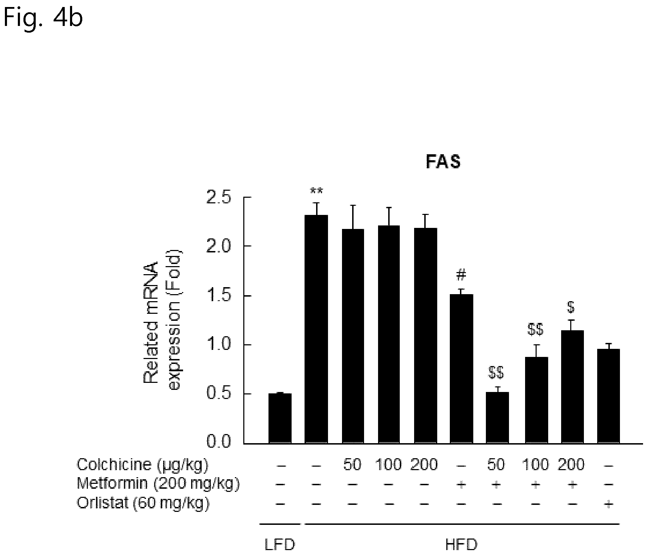

Meanwhile, after the drugs were administered to the obesity-induced rats, the expression of obesity-related genes SREBP-1c and FAS was measured, and the results thereof were illustrated in FIGS. 4A and 4B.

According to FIG. 4, in the epididymal fat of the obesity-induced rats by the HFD, it was confirmed that the expression of the obesity-related genes SREBP-1c and FAS was not changed by alone-administration of colchicine and slightly reduced by alone-administration of metformin. In comparison, in co-administration of colchicine and metformin, it was confirmed that the expression of SREBP-1c and FAS was much reduced in the epididymal fat of the obesity-induced rats. Particularly, it was confirmed that when the content of colchicine was smaller than the content of metformin, the expression of the genes was relatively much reduced, and much more reduced than the control drug, Xenical.

Finally, after the drugs were administered to the obesity-induced rats, changes in blood levels of aspartate aminotransferase (AST) and alanine transaminase (ALT) were measured and illustrated in Table 1 below.

As illustrated in Table 1, it was confirmed that the blood levels of AST and ALT increased by the HFD were not changed by alone-administration of colchicine and slightly reduced in alone-administration of metformin. In comparison, in co-administration of colchicine and metformin, it was confirmed that the blood levels of AST and ALT were much reduced, and it was confirmed that when the content of colchicine was smaller than the content of metformin, the levels were much reduced and much more reduced than the control drug, Xenical.

**[Table 1]**

| **Group** | **ALT (U/L)** | **AST (U/L)** |
|---|---|---|
| ND | 32.55 ± 1.03 | 35.41 ± 1.51 |
| HFD | 305.16 ± 2.05 | 408.58 ± 1.36 |
| HFD + Colchicine (50 µg/kg) | 175.08 ± 2.78 | 372.26 ± 3.75 |
| HFD + Colchicine (100 µg/kg) | 164.5 ± 2.11 | 346.91 ± 3.90 |
| HFD + Colchicine (200 µg/kg) | 157.38 ± 3.3 | 309.24 ± 2.61 |
| HFD + Metformin (200 mg/kg) | 156.41 ± 2.77 | 284.37 ± 2.78 |
| HFD + Metformin (200 mg/kg) + Colchicine (50 µg/kg) | 54.98 ± 1.84 | 61.44 ± 2.65 |
| HFD + Metformin (200 mg/kg) + Colchicine (100 µg/kg) | 92.45 ± 1.25 | 127.63 ± 3.27 |
| HFD + Metformin (200 mg/kg) + Colchicine (200 µg/kg) | 125.50 ± 3.43 | 153.41 ± 3.42 |
| HFD + Orlistat (60 mg/kg) | 175.20 ± 3.11 | 203.31 ± 3.72 |

As seen from the results, there was almost no effect of reducing the fat of the obesity-induced rats by alone-administration of colchicine, and there was a slight effect by alone-administration of metformin, but the effect was significantly shown by co-administration of colchicine and metformin. This means that the two drugs exhibit an apparent synergic effect to efficiently prevent and treat the obesity, and the pharmaceutical composition of combining colchicine and metformin provided in the present invention may be used as an excellent pharmaceutical composition for treating or preventing the obesity.

The dose of the pharmaceutical composition provided in the present invention may be determined by those skilled in the art by considering the purpose of use, the addiction of the disease, the age, body weight, gender, and medical history of the patient, the type of a material used as an effective agent, etc. The pharmaceutical composition of the present invention may be administered in an amount of 20 to 1000 mg/kg, more preferably 50 to 400 mg/kg a day with respect to the body weight of a mammal including the human, and the administration frequency of the pharmaceutical composition of the present invention is not particularly limited, but the pharmaceutical composition may be administered once to three times a day or administered several times by dividing the dose.

The aforementioned description of the present invention is used for exemplification, and it can be understood by those skilled in the art that the present invention can be easily modified in other detailed forms without changing the technical spirit or requisite features of the present invention. Therefore, it should be appreciated that the aforementioned embodiments are illustrative in all aspects and are not restricted.

### [Industrial Applicability]

The present invention is industrially applicable.

## Claims

1. A pharmaceutical composition for use in preventing and/or treating adipositas comprising both colchicine and metformin.

2. The pharmaceutical composition for use in preventing and/or treating adipositas of claim 1, wherein the content of the colchicine included in the pharmaceutical composition is 5 to 300 µg/kg and the content of the metformin is 50 to 400 mg/kg.

3. The pharmaceutical composition for use in preventing and/or treating adipositas of claim 1, wherein the content of the colchicine included in the pharmaceutical composition is 50 to 200 µg/kg and the content of the metformin is 100 to 200 mg/kg.

4. The pharmaceutical composition for use in preventing and/or treating adipositas of claim 1, wherein a content ratio of the colchicine and the metformin included in the pharmaceutical composition is 1:1,000 to 1:20,000 as the content of the metformin to the content of the colchicine.

5. The pharmaceutical composition for use in preventing and/or treating adipositas of claim 1, wherein a content ratio of the colchicine and the metformin included in the pharmaceutical composition is 1:2,000 to 1:5,000 as the content of the metformin to the content of the colchicine.

6. The pharmaceutical composition for use in preventing and/or treating adipositas of any one of claims 1 to 5, wherein the pharmaceutical composition is prepared by any one formulation selected from the group consisting of powders, granules, tablets, capsules, suspensions, emulsions, syrups, aerosols, external preparations, suppositories, and injectable solutions.

7. The pharmaceutical composition for use in preventing and/or treating adipositas of any one of claims 1 to 5, wherein the pharmaceutical composition further comprises one or more ingredients selected from the group consisting of lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, and magnesium stearate.

8. The pharmaceutical composition for use in preventing and/or treating adipositas of any one of claims 1 to 5, wherein the pharmaceutical composition further comprises one or more ingredients selected from the group consisting of thiamine, riboflavin, niacin, pantothenic acid, pyridoxine, cobalamin, vitamin C, vitamin D, vitamin E, and N-acetylcysteine.
